# EUROPEAN PATENT SPECIFICATION

(11) **EP 1 038 145 B1**
(45) Date of publication and mention of the grant of the patent: **09.11.2005**
(21) Application number: 98941953.6
(22) Date of filing: 09.09.1998
(51) Int. Cl.: F25D 23/08, A47B 96/00, B29C 51/42, B29C 53/04, B29C 53/84, B29C 65/18, F25D 23/06, B29C 69/00, B29C 49/00, B29C 65/20

(54) **A CABINET AND A WALL MEMBER**
SCHRANK UND WANDTEIL
ARMOIRE ET ELEMENT DE PAROI

(30) Priority: 09.09.1997 NZ 32870397
(43) Date of publication of application: 27.09.2000
(73) Proprietor: Fisher & Paykel Appliances Limited, East Tamaki, Auckland (NZ)
(72) Inventor: VAUGHAN, Stewart, Auckland (NZ); WITTEN-HANNAH, Daniel, Auckland (NZ)
(74) Representative: Brown, John D.
(86) International application number: PCT/NZ1998/000134
(87) International publication number: WO 1999/013280

(56) References cited:
- EP-A- 0 769 667
- EP-A2- 0 839 633
- WO-A-90/07457
- WO-A-92/14603
- WO-A-97/45326
- AU-A- 3 636 078
- GB-A- 2 085 797
- US-A- 3 142 406
- US-A- 3 615 149
- US-A- 4 006 947
- US-A- 5 168 621
- US-A- 5 273 801
- US-A- 5 374 118
- PATENT ABSTRACTS OF JAPAN vol. 015, no. 208 (M-1117), 28 May 1991 (1991-05-28) & JP 03 056238 A (SHOWA DENKO KK), 11 March 1991 (1991-03-11)
- DERWENT ABSTRACT, Accession No. 97-276037/25, Class A32; & JP 09099483 A (TSUTSUNAKA PLASTIC IND CO LTD) 15 April 1997.

## Description

### TECHNICAL FIELD

This invention relates to a cabinet and a wall member formed by twin sheet thermoforming.

A cabinet according to the preamble of claim 1 and a wall member according to the preamble of claim 4 are known from document US 5 374 118.

### BACKGROUND ART

At present the construction of domestic refrigerators, particularly the cabinets, are commonly made from a formed steel exterior, which is either pre-painted or post-painted, into which a thermoformed plastic interior is placed locating in a roll formed edge. A steel or plastic base is attached to the bottom and top of this assembly along with a back panel of similar materials to create a closed cavity between the exterior and interior. This assembly is placed into a jig that supports the walls of the cabinet whilst insulation is injected and expanded into the cavity. Associated with this type of construction are additional processes that seal the joints between the individual parts in order to prevent the expanded foam from leaking.

US Patent 4,580,852 shows a refrigerator incorporating a cabinet of this type. The major drawbacks with this type of construction and manufacturing process are the number of individual parts, processes, and their associated costs. In addition to this the processes are time consuming and labour intensive.

US Patent 5,374,118 assigned to Whirlpool Corporation shows a refrigerator cabinet wherein the side walls and top are formed by twin-wall thermoforming as a single part. They are folded and a back is fitted to the cabinet to support them in position. The back of the cabinet incorporates the refrigeration system. While this system offers a significant improvement over the prior art, difficulties are perceived with the overall cabinet space occupied by the refrigeration system carrying back unit and with difficulties of connecting and sealing the back unit to the remainder of the refrigerator cabinet.

### DISCLOSURE OF THE INVENTION

It is therefore an object of the present invention to provide a cabinet and/or parts thereof and/or a method of forming same which will go some way towards overcoming the above disadvantages or will at least provide the public with a useful choice.

In one aspect the invention consists in a cabinet for an appliance including a wall member formed by twin sheet thermoforming, said wall member incorporating a plurality of panels, said panels connected to one another by one or more joined edges where said twin sheets contact to form a seam, and said wall member being in a folded condition along one or more of said seams such that said plurality of panels form a plurality of walls of said cabinet, being, as a group, differently configured to when said wall member was initially thermoformed, wherein at least one of said plurality of panels includes a transition, which in the folded condition, forms a corner of the cabinet, characterized in that one or more of said folded seams do not coincide with the corner between walls of said cabinet that correspond to the panels joined by the respective folded seam.

In a still further aspect the invention consists in a wall member formed by twin sheet thermoforming including one or more seams where said twin sheets contact, said wall member adapted to be folded at one or more of said seams to form a plurality of walls, at least one said seam being intended to be folded through a set angle in use in assembling a cabinet, and at least one said sheet of said twin sheets including at least a first and a second surface, characterised in that, at or substantially at said seam, a transition between said first and second surfaces is formed the angle of said being the complement of the said set angle, such that if said seam is folded through said set angle, the first and the second surface will become aligned across the seam to form a substantially containous plane on the outside of said cabinet.

To those skilled in the art to which the invention relates, many changes in construction and widely differing embodiments and applications of the invention will suggest themselves without departing from the scope of the invention as defined in the appended claims.

### BRIEF DESCRIPTION OF THE DRAWINGS

Embodiments will now be described in detail with reference to the accompanying drawings in which;
Figure 1 is a cross-sectional end elevation through a thermoforming mould for forming the twin sheet wall forming panel, in a closed configuration,
Figure 2 is a cross-sectional side elevation on A-A of the mould of Figure 1 during a foaming operation,
Figure 3 is a perspective view of the twin sheet panel of a first embodiment subsequent to removal from the mould,
Figure 4 is a partial perspective view in cross-section of an end edge of the panel immediately after removal from the mould,
Figure 5 is an exploded perspective view of a refrigerator cabinet assembly,
Figure 6 is a perspective view in cross section of a twin sheet panel according to an alternative embodiment,
Figure 7 is an exploded perspective view of a refrigerator cabinet assembly according to said alternative embodiment,
Figure 8 is a cross sectional plan elevation through the portion of a twin sheet panel according to the preferred embodiment of the present invention that, when folded up to form a refrigerator cabinet assembly, will form a corner between the back and a side thereof, and additionally showing a tool for heating and softening parts of one or both of the twin sheets, and
Figure 9 is a cross sectional plan elevation through the refrigerator cabinet corner formed from that panel portion of Figure 8.

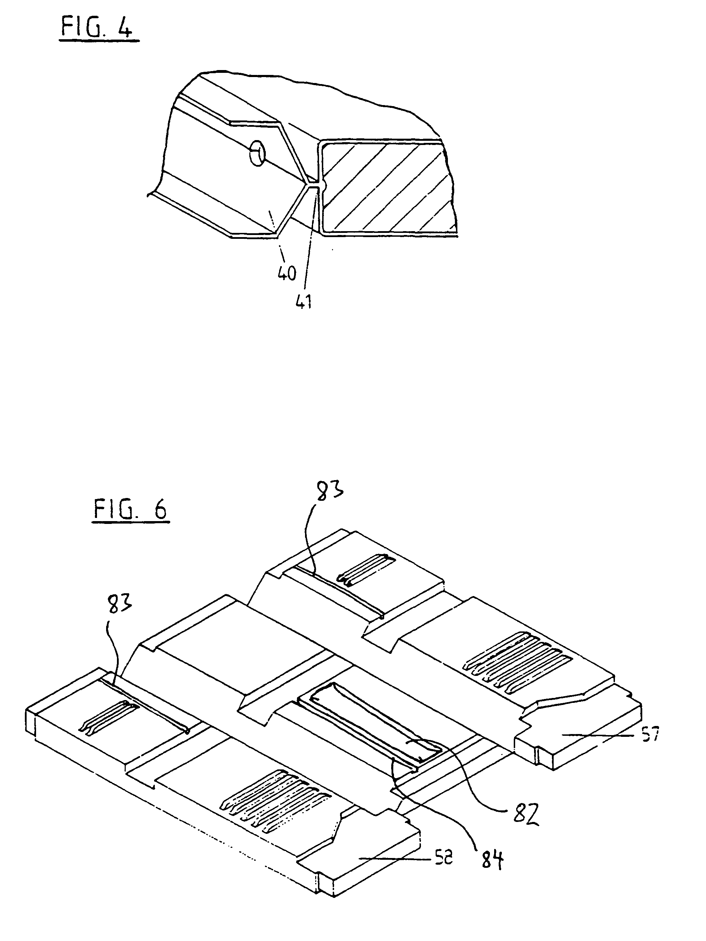

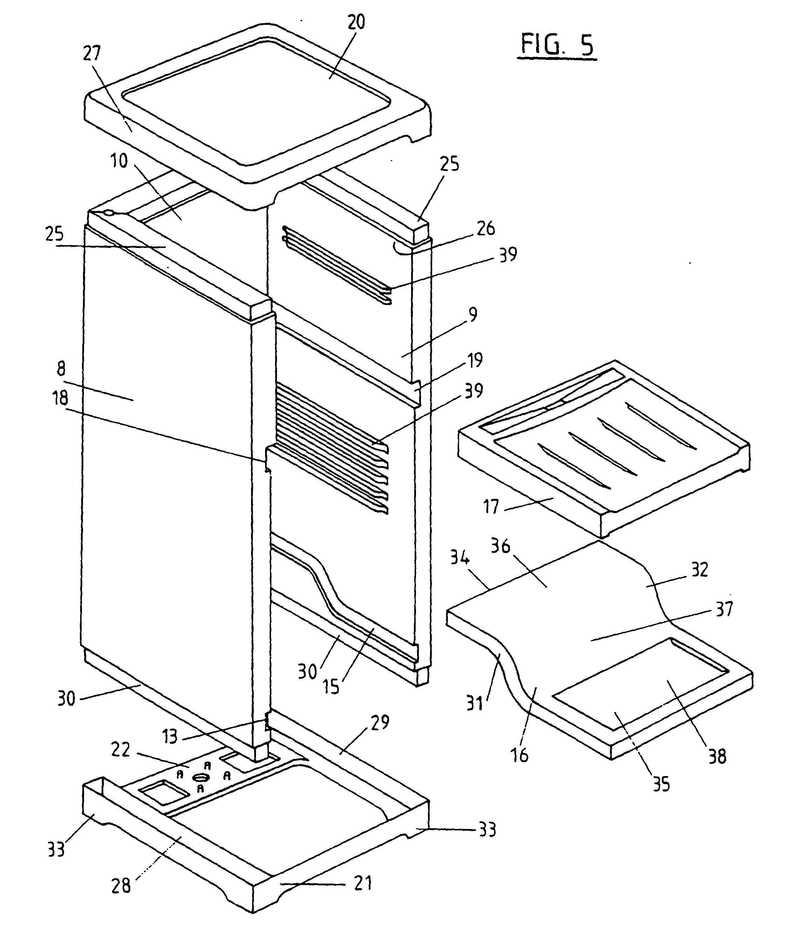

The embodiments shown in Figures 4 to 7 and described in the corresponding description do not fall within the scope of the claims.

### BEST MODES FOR CARRYING OUT THE INVENTION

With reference to Figure 5, a cabinet is shown for a refrigerator which has side walls 8,9 and a rear wall 10 which are integrally connected at hinged regions 6. The side walls 8,9 and rear wall 10 are folded at the hinged regions to form the sides 8,9 and rear wall 10 as shown. The walls 8,9,10 are retained in the folded condition by a top cap 20 and by a base plinth 21. The top cap also serves to enclose the top end of the refrigeration chamber defined by the side walls 8,9 and back wall 10. A floor partition 16 is disposed within the chamber to enclose the bottom side of the refrigeration chamber. The base plinth 21 is disposed with connections 22 for mounting elements of the refrigeration system, notably the compressor, control system and expansion valve. The base plinth 21 may otherwise remain substantially open. For refrigerator configurations incorporating a fresh food refrigeration compartment and a freezer compartment, a compartment divider 17 is provided.

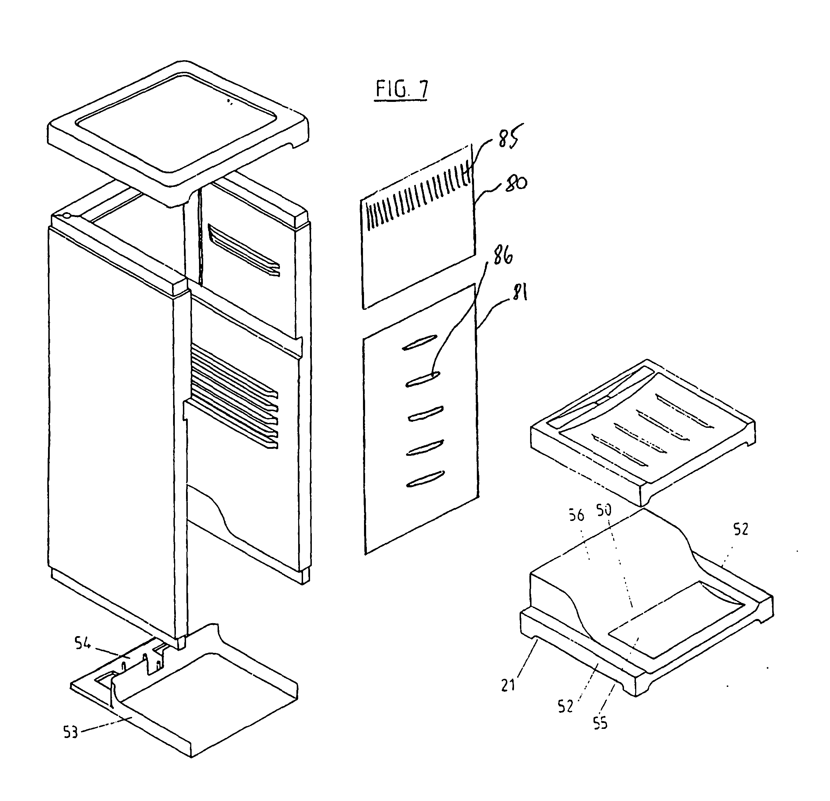

With reference to Figure 7 an alternative embodiment is shown wherein the bottom partition is incorporated in the base plinth in an integrated manner as a floor member 50. The floor member 50 defines a space there below bordered by side footings 52. Refrigeration system mechanical components are located on connections 54 of a carrying tray 53 which is slidably fitted within the space below the floor member 50 and supported by the side footings 52.

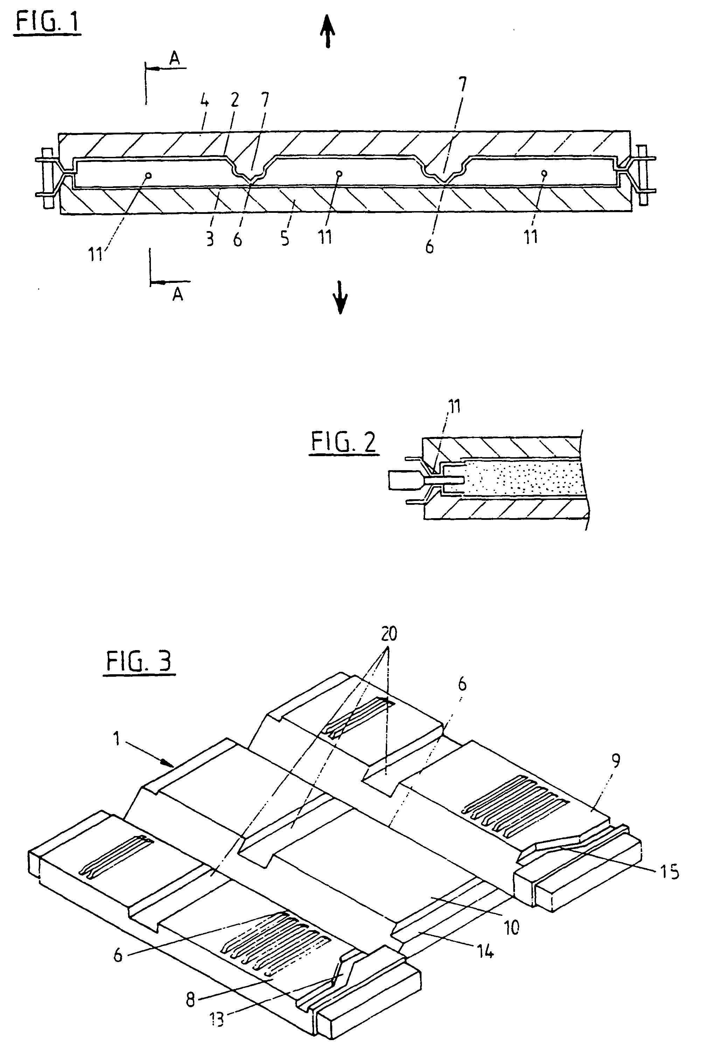

The method of forming the side and back walls 8,9,10 will be described later with reference to Figures 1-4, as will the overall assembly of the refrigerator.

With reference to Figures 5 and 7 the top cap 20 of the refrigerator both encloses the top end of the refrigeration space and also retains the top edge 25 of the walls 8-10 within a channel formed on the lower face thereof. The top edge 25 of the walls 8-10 is preferably formed with a rebate 26, thereby forming a tongue-like projection, so that when fitted, the outer side faces and end face 27 of the top cap 20 sit flush with the external surfaces of the side and rear walls 8-10.

Similarly the base plinth 21 is provided with channels 28 and 29 adjacent the side extremities thereof. The channels 28, 29 accommodate tongue portions 30 of the lower edge of the side walls 8 and 10. Once more the tongued or rebated configuration allows the side faces of the base plinth to sit flush with the side surfaces of the refrigerator cabinet. As can be seen from Figure 5 in conjunction with Figure 3, the rear wall 10 of the cabinet preferably finishes at a point above the lower edge of the side walls 8 and 9. This leaves an opening at the rear of the refrigerator for access to the refrigeration system components (mounted on the base plinth connections 22 in figure 5 and on support tray 51 in figure 7), and allows air flow therearound as necessary. The base plinth 21 also preferably includes foot portions 33 extending slightly downwardly therefrom to support the refrigerator cabinet on a supporting surface. Adjustable feet as are known in the prior art may be fitted as required.

With reference to Figure 5 floor partition 16 is preferably disposed with the side edges 31, 32 thereof disposed within channels 13, 15 formed in the interior side walls of the refrigerator cabinet and the rear edge 34 thereof disposed in recessed channel 14 along the lower edge of the rear wall. The floor preferably includes a transition from a substantially horizontal forward portion 35 to a substantially horizontal rearward portion 36 via a sloped and curved transition region 37. The floor partition may also include interior detailing such as drip tray 38.

With reference to Figures 6 and 7, in the alternative embodiment the floor preferably includes a similar transition between a front plateau 55 and a rear plateau 56. The interior faces of the side walls of the refrigerator cabinet are formed with a shaped rebate 57,58 therein, the upper edge of which generally follows the contour of the upper lateral edges of said floor member, with the raised part of said base plinth being located within the rebate 57,58.

With reference to Figures 5 and 7 the walls 8, 9 and rear wall 10 of the cabinet may have their internal faces thereof formed with detailing such as tray supporting rails 39 formed integrally therein. Other manners of applying such details to these walls may also be apparent to persons skilled in the art, for example, by plastic welding facia panels to flat surfaces, however this seen as a rather inefficient manner of forming such detailing.

Similarly the divider 17 may contain additional details for door hinging, air ducting and interior styling.

With reference to Figures 1 and 3 the side walls 8,9 and rear wall 10 of the cabinet are formed by simultaneously thermoforming the exterior and interior of the cabinet as a single flat panel 1 from two plastic sheets 2,3 that can be of differing thicknesses, colours or material composition. The sheets 2, 3 are heated and are initially held at their periphery by supporting flanges 12. Air is injected between the sheets 2,3 as the moulds, interior 4 and exterior 5, are closed over the sheets 2,3 assisting in the thermoforming process. The above aspects of the twin-sheet thermoforming process itself is known in the art and will not be described in detail herein.

The correspondingly formed panel 1 has two folding/hinging regions 6 placed into it by ridges 7 in the profile of the interior mould 4. The hinging regions 6 divide the panel into three cavities, two side walls 8,9 and a rear wall 10. In the hinging region the interior sheet 4 is attached to the exterior sheet 5 to form the folding line.

With reference to Figure 4, after thermoforming the panel remains in the mould and insulating foam 40 is injected into the three cavities through are injection holes 11 from the thermoforming process. The thermoforming mould 4,5 then acts at a foaming jig, supporting the walls whilst the foam expands and sets.

After sufficient time for the foam to cure, the foamed panel is then removed from the mould 4,5 and the supporting flanges 12. With reference to Figure 4, during removal from the mould or some time thereafter and before assembly, excess material 40 is trimmed from the moulded member 1. The material is preferably trimmed flush with the join 41 between internal and external sheets, to leave a clean exterior finish. Exposed joins may be finely polished to provide an aesthetic finish.

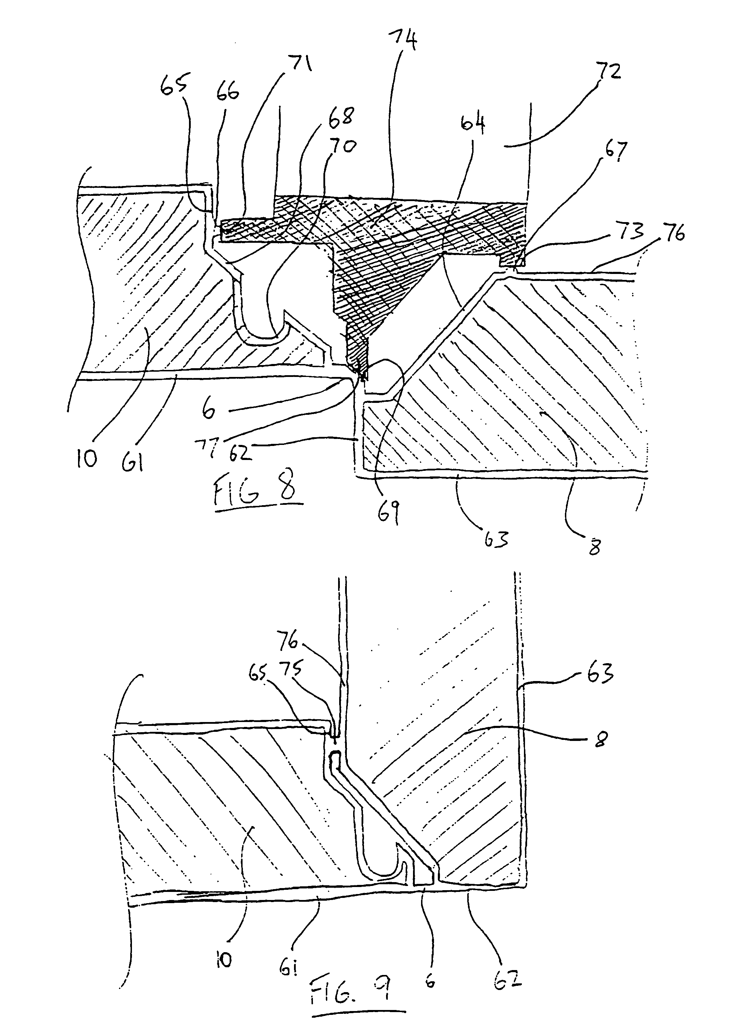

Referring to Figure 8 an advantageous configuration of the corner area of a the thermoformed panel is shown. The corner area is between a side wall 8 and the rear wall 10 of the refrigerator, with the twin sheets essentially being assigned as either an inner sheet or an outer sheet, depending on whether they form the inner faces of the corner or the outer faces. The corner is intended to be formed by folding the panel through a set angle at the seam 6 where the inner and outer sheets contact, the angle corresponding to the intended angle between the walls when assembled. To this end the regions 64, 68 of the inner sheet approaching the seam include an angle substantially the same as the set angle, so that when the panel is folded at the seam these regions will be substantially adjacent and there will not be a significant opening or crevice there between. Configurations are of course possible where this included angle is biassed toward one sheet or another, with a seam with width approximately the same as the thickness of the panel being one extreme possibility. However in the preferred embodiment, for better overall strength and ease of forming, the included angle is embodied as a pair of faces 64, 68 which are at a general angle of 45 degrees to the general plane of the sheet, to thereby mutually include an angle of approximately 90 degrees.

The outer sheet is configured to with a transition at the seam of the thermoformed panel, between the outer face 61 of the panel section 8 that will broadly form the back wall, and the minor outer face 62 of the panel section 10 that will broadly form the side wall, which is basically the complement of the set angle through which the panel is to be folded at the seam 6. With the outer sheet thus configured, when the sheet is folded through the set angle the face 61 and the face 62 will become aligned across the seam to form a substantially continuous plane, the back wall surface. There is of course a further transition in the outer sheet, falling in that part of the panel that will form the side wall, and the transition being between the minor face 62 and the major face 63 of that panel section, with that transition, when folded, forming the corner of the cabinet. In this way the seam between the side and rear panels of the cabinet is provided along a line that is parallel with and near the corner, but is not at the corner, and so can be hidden on the back face of the refrigerator, which in nearly all installations will be fairly well hidden from view. This is depicted in Figure 9 which shows the panel in its folded configuration, the seam 6 being clearly disposed on the back face of the refrigerator cabinet. Other configurations of these transitions are of course possible depending on the angles through which the panel is to be folded, possible overall curvatures of the panel sections and possible curvature of the corner itself, without departing from the general intention of this aspect of the invention.

With continued reference to Figures 8 and 9 aspects of the preferred folding process are depicted. In the prior art systems that have suggested folding twin sheet panels at a seam, the seam has been heated by disposing a radiant heater, such as a wire of high electrical resistance, in close proximity to the seam immediately prior to folding, to soften the seam. However with a panel of the size necessary to form the walls of a refrigerator cabinet it has been found that such techniques are inappropriate, for example due to wire sag, and the ability to hold the heater sufficiently close to heat the seam without also heating and thereby facilitating deformation of the surrounding sheet. Therefor in the preferred assembly method a heater is provided in which a highly conductive heater head 74 is supported on a heater support member 72, which is moveable relative to the panel to bring a heating face 77 thereof into contact with the seam 6 of the panel. The heater head and heating face are preferably substantially coextensive with the seam 6. To facilitate this contact the seam region may be formed having a significant width, and may include a greater heat receiving surface, such as by the inclusion of chamfer 69 on the inner side thereof. This heating operation may advantageously be provided while the panel is still supported on its lower mould, however it may equally be carried out with the panel support ed on another supporting surface so long as it is reasonably accurately positioned with respect to the heater head 74 and head support member 72. It should be readily apparent that the use of a conductive heater head allows for a more localised heating, particularly as the conductive head is more readily formed from a solid and rigid material, such as an aluminium block. This lessens the adverse heating that is caused to adjacent areas of the sheet and reduces the heating time. The heat conductive block preferably includes a coating which helps the block release from the heated plastic material without deforming the plastic material. A PTFE coating has been found suitable for this purpose.

As already described the inner sheet of the panel includes an angle between the faces 68 and 64 which are to eventually nearly meet in the assembled construction. This similar angle also exists between at least the regions of faces 65 and 76 which are adjacent the faces 68 and 64, but further from the seam 6. Due to the step that is advantageously incorporated in the outer sheet (for reasons described above), these regions of the faces 65 and 76 will also come into close alignment and proximity. In the preferred embodiment of the invention the close proximity and alignment of the faces, 68 and 64 and/or 65 and 76, is used to provide a further fused joining between the panel sections, spaced away from the seam 6, to thereby permanently lock the panels into their folded configuration. To assist the fusing process nodules 66, 67 of plastic material are raised from the general surrounding surfaces 76, 65. The nodules may comprise localised raised areas, or may comprise ridges being substantially coextensive with the seam 6. The conductive heating head 74 is provided with further heating faces 61 and 73 and the heater support 72 is configured to bring these heating faces into contact with the nodules, 66 and 67 respectively, simultaneously with contacting face 77 against the seam 6. With the panel being subsequently folded while the nodules are substantially molten the nodules contact and fuse together to form a weld 75 between the faces 65 and 76 of the inner sheet of the panel. In use the weld not only provides a significant increase in the stiffness of the cabinet but also serves to seal the crevice between the faces 68, 64 of the inner sheet at a location near the surface of the crevice.

The corner is shown to be between the left side 8 and back 10 walls of the refrigerator cabinet but it is clearly applicable to other seams between sections of a thermoformed panel that are intended to be folded in formation of an article such as a cabinet, particularly where one of the external faces thereof is likely to be substantially hidden from view in general use.

In the embodiment of Figure 5 floor partition 16 is introduced into the assembly prior to the folding and is locked in place by a recess 13, 14, 15 formed in the side and back walls of the folded panel once the side walls 8, 9 have been fully folded into position. The floor 16 is preferably a foam core sandwich with a plastic outer skin.

With reference to Figures 5 and 7 for dual temperature fridges a compartment divider 17 of similar construction to the floor 16 is inserted into channels 18, 19 formed in the side walls, as recess 20 extending across the panel (see Figure 3) to create the division between the freezer and refrigerator compartments. This divider 17 interlocks the sides 8, 9 of the folded panel 1 and is in turn locked in place by the restrained sides of the folded panel. The divider 17 contains additional details for door hinging, air ducting, and interior styling. The divider may be provided with a dove tail, mortise and tenon, connection to said side walls or may be provided with simple rectangular side edges.

With the divider and/or floor partition in place the top cap 20 is fitted to the top edge 25 of the folded panel. The tongue portion formed by rebate 26 fits within a channel in the underside of the top cap 20, to be retained thereby in the folded configuration.

The folded panel is further fitted with the base plinth 21 at the bottom edge thereof with the lower edge 30 of side walls 8 and 9, having the tongued portion thereof fitting within channels 28, 29 formed on the top face along the sides of the base plinth. The base plinth 21, like the top cap 20 retains the wall forming panel 1 in the folded configuration shown in Figures 5 and 7.

With further reference to Figure 7, some additional detail demonstrating how a refrigeration system may be incorporated in the refrigerator can be seen. In particular the refrigerator includes a baffle panel 80, 81 in each of the freezer and refrigerator compartments respectively, with each baffle panel forming the back wall of its respective compartment. Air recirculation ducts, which may be partially formed (for example the channel 82) in the twin sheet thermoformed panel, are concealed behind the baffles. The baffle for the freezer compartment is configured to be spaced forward of the inner face of the thermoformed panel, with its edges secured in grooves 83 in the side walls of the compartment, to provide a subcompartment therebehind. This subcompartment is utilised for housing the evaporator of the refrigeration system. One or more fans are positioned in the ducts which force the recirculation of air over the outer surface of the evaporator, with the cooled air then being passed into either the freezer or refrigeration compartment as desired, through the apertures 85, 86 in the respective baffle 80, 81. A channel 84 may be provided in the thermoformed panel to facilitate the egress of condensation which may form from time to time on the evaporator and be removed during a defrosting process. The channel 84 leads to a position just above the refrigerator compressor, where the condensation may fall into a tray mounted on the compressor head to thereby absorb heat from the compressor head and be evaporated away. Refrigeration systems of this general configuration form are known in the art, and do not form a significant part of the present invention.

A refrigeration system as is known in the art, such as a standard vapour compression refrigeration system may be fitted to the cabinet. In such a system the compressor, condenser and any control system may be carried by the base plinth with the evaporator inside the refrigeration space, such as in the manner described above with reference to Figures 6 and 7. A door or doors is mounted on the fridge front, supported between hinge parts on the front edges of the top cap and base plinth and on the compartment divider panel. The doors are preferably also formed by twin sheet thermoforming with insulating foam injected into the cavity, as is known in the art.

The above description has been given with reference to refrigerator cabinets. The process is thought to be sufficiently versatile to also be appropriate for other cabinet types, particularly cabinets requiring a twin walled insulated structure. It is also readily apparent that while the above cabinet has been described as a pair of side walls and a rear wall, it would be a simple modification to incorporate any number of walls, with a corresponding change in the included angle between abutting transition faces.

It is readily apparent that the refrigerator cabinet and as described above, includes significant advantages over the prior art. The refrigerator cabinet is provided with a simple construction having few parts, and which parts fit together in a manner so as to be self interlocking, giving the cabinet strength and rigidity. Cabinet dividers and partitions are engaged within the cabinet in an interlocking manner which provides good seals from both a thermal and moisture perspective, as does the engagement of the top cap with the top edge of the folded wall assembly. The base plinth, provided with the many mechanical elements of the refrigerator system is disposed beneath the bottom partition, easily accessible from the rear of the fridge, and minimising wasted potential refrigeration space within the refrigerator cabinet. It will also be appreciated that smooth curves and exterior lines will be readily possible, moulded into the exterior shape of the top cap, and into the outer sheet of the folded panel.

It will also be readily appreciated that the simplicity of assembly means the refrigerator cabinet can be shipped in a knockdown form, with final assembly taking place at a n entirely separate and potentially distant location than component production. This will provide significant benefits in shipping costs, where costs for low density products (such as refrigerators) are usually based on volume rather than weight.

## Claims

1. A cabinet for an appliance including a wall member formed by twin sheet thermoforming, said wall member (1) incorporating a plurality of panels, said panels connected to one another by one or more joined edges where said twin sheets (2, 3) contact to form a seam (6), and said wall member (1) being in a folded condition along one or more of said seams (6) such that said plurality of panels form a plurality of walls (8, 9, 10) of said cabinet, being, as a group, differently configured to when said wall member was initially thermoformed, wherein at least one of said plurality of panels includes a transition, which in the folded condition, forms a corner of the cabinet, **characterized in that** one or more of said folded seams (6) do not coincide with the corner between the walls of said cabinet that correspond to the panels joined by the respective folded seam (6).

2. A cabinet as claimed in claim 1 wherein said respective folded seam (6) is between an edge of one panel which forms an edge of the surface (61) at least contributing to one said wall of said corner of said one panel, and the edge of another panel which forms an edge of a surface (62) at an angle to the surface of said other panel at least contributing to the other wall of said corner, said wall member (1) folded at said seam (6), through said angle, such that said surface (62) at an angle of said other panel contributes to said one said wall of said corner, and separates said folded seam (6) from said corner.

3. A cabinet as claimed in claim 1 wherein each said respective folded seam (6) is between an edge of one panel and an edge of another panel, and with said wall member in an unfolded condition said one panel includes an exterior wall surface (61) adjacent said seam, and said other panel includes a first exterior wall surface (62) and a second exterior wall surface (63), said first exterior wall surface (62) being adjacent said seam and at an angle to said exterior wall surface (61) of said one panel, and said wall member (1) is in said folded condition with said exterior wall surface (61) of said one panel and said exterior wall surface (62) of said other panel contributing to one wall of said cabinet, and said first exterior wall (62) surface of said other panel separating said folded seam (6) from said corner.

4. A wall member formed by twin sheet thermoforming including one or more seams (6) where said twin sheets contact, said wall member adapted to be folded at one or more of said seams (6) to form a plurality of walls, at least one said seam (6) being intended to be folded through a set angle in use in assembling a cabinet, and at least one said sheet of said twin sheets including at least a first (61) and a second surface (62), **characterised in that**, at or substantially at said seam (6), a transition between said first and second surfaces is formed, the angle of said transition being the complement of the said set angle, such that if said seam is folded through said set angle, the first surface (61) and the second surface (62) will become aligned across the seam to form a substantially continuous plane on the outside of said cabinet.

5. A wall member as claimed in claim 4 wherein said sheet having said first and second surfaces includes a third surface (63), and said second surface (62) meets said third surface (63) at a projecting corner substantially parallel to said seam (6) but spaced therefrom, said corner including said set angle such that said third surface (63) is substantially co-planar with said first surface.

6. A wall member as claimed in claim 5 including a pair of said seams (6), parallel and spaced apart, dividing said wall member into three side-by-side panels, one sheet of said wall member having said first, second and third surfaces, and fourth and fifth surfaces, with each of said first, third and fifth surfaces constituting a substantially planar major surface associated with a respective panel, said first, third and fifth surfaces being substantially parallel, with said pair of seams (6) being at opposed edges of said first surface and said second surface extending from the seamward edge of said third surface to the respective seam edge of said first surface and said fourth surface extending from the seamward edge of said fifth surface to the respective seam edge of said first surface, and second and fourth surfaces being approximately 90 degrees to said first, third and fifth surfaces, such that if said seams (6) are folded through 90 degrees, said second and fourth surfaces will align with said first surface and said third and fifth surfaces will extended perpendicularly from the edges of said second and fourth surfaces with the folded seams (6) disposed entirely on a face formed by said first, second and fourth surfaces.

## Patentansprüche

1. Schrank für ein Haushaltsgerät, umfassend ein Wandelement, welches durch Doppellagen-Thermoformen ausgebildet ist, wobei das Wandelement (1) eine Mehrzahl von Platten umfasst, wobei die Platten miteinander durch einen oder mehrere verbundene Ränder dort, wo die Doppellagen (2, 3) sich berühren, derart verbunden sind, dass sie einen Falz (6) bilden, und wobei sich das Wandelement (1) in einem geklappten Zustand entlang eines oder mehrerer der Falze (6) befindet, sodass eine Mehrzahl von Platten eine Mehrzahl von Wänden (8, 9, 10) des Schranks bildet, welche als eine Gruppe anders konfiguriert sind, als dann, als das Wandelement anfangs thermogeformt wurde, wobei wenigstens eine aus der Mehrzahl von Platten einen Übergang umfasst, welcher in dem geklappten Zustand eine Ecke des Schranks bildet, **dadurch gekennzeichnet, dass** einer oder mehrere der geklappten Falze (6) nicht mit der Ecke zwischen denjenigen Wänden des Schranks übereinstimmen, welche den Platten entsprechen, die durch den jeweiligen geklappten Falz (6) verbunden sind.

2. Schrank nach Anspruch 1, wobei der jeweilige geklappte Falz (6) zwischen einem Rand einer Platte, welche einen Rand der Fläche (61) bildet, die wenigstens zu einer besagten Wand der Ecke der einen Platte beiträgt, und dem Rand einer weiteren Platte sich befindet, welche einen Rand einer Fläche (62) in einem Winkel zu der Fläche der anderen Platte bildet, die wenigstens zu der anderen Wand der Ecke beiträgt, wobei das Wandelement (1) bei dem Falz (6) um den Winkel derart geklappt ist, dass die Fläche (62) in einem Winkel der anderen Platte zu der einen Wand der Ecke beiträgt und den geklappten Falz (6) von der Ecke trennt.

3. Schrank nach Anspruch 1, wobei jeder jeweilige geklappte Falz (6) zwischen einem Rand einer Platte und einem Rand einer weiteren Platte sich befindet, wobei die eine Platte dann, wenn das Wandelement in einem ausgeklappten Zustand ist, eine Außenwandfläche (61) umfasst, welche dem Falz benachbart ist, und die andere Platte eine erste Außenwandfläche (62) und eine zweite Außenwandfläche (63) umfasst, wobei die erste Außenwandfläche (62) dem Falz benachbart ist und in einem Winkel zu der Außenwandfläche (61) der einen Platte sich befindet, und das Wandelement (1) sich in dem geklappten Zustand befindet, wobei die Außenwandfläche (61) der einen Platte und die Außenwandfläche (62) der anderen Platte zu einer Wand des Schranks beitragen, und wobei die erste Außenwandfläche (62) der anderen Platte den geklappten Falz (6) von der Ecke trennt.

4. Wandelement, welches durch Doppellagen-Thermoformen ausgebildet ist, welches einen oder mehrere Falze (6) dort umfasst, wo sich die Doppellagen berühren, wobei das Wandelement dazu ausgebildet ist, an einem oder mehreren der Falze (6) geklappt zu werden, um eine Mehrzahl von Wänden zu bilden, wobei wenigstens ein besagter Falz (6) bei der Verwendung bei der Montage eines Schranks um einen eingestellten Winkel geklappt werden soll und wenigstens eine besagte Lage der Doppellagen wenigstens eine erste (61) und eine zweite Fläche (62) umfasst, **dadurch gekennzeichnet, dass** bei oder im Wesentlichen bei dem Falz (6) zwischen der ersten und der zweiten Fläche ein Übergang ausgebildet ist, wobei der Winkel des Übergangs das Komplement des eingestellten Winkels ist, sodass dann, wenn der Falz um den eingestellten Winkel geklappt wird, die erste Fläche (61) und die zweite Fläche (62) über den Falz hinweg ausgerichtet werden, um eine im Wesentlichen fortlaufende Ebene an der Außenseite des Schranks zu bilden.

5. Wandelement nach Anspruch 4, wobei die Lage mit der ersten und der zweiten Fläche eine dritte Fläche (63) umfasst, und die zweite Fläche (62) die dritte Fläche (63) bei einer vorragenden Ecke trifft, welche im Wesentlichen parallel zu dem Falz (6) ist, aber von diesem mit Abstand angeordnet ist, wobei die Ecke den eingestellten Winkel einschließt, sodass die dritte Fläche (63) im Wesentlichen mit der ersten Fläche komplanar ist.

6. Wandelement nach Anspruch 5, umfassend ein Paar der Falze (6), welche parallel und mit Abstand voneinander angeordnet sind, das das Wandelement in drei Platten Seite an Seite teilt, wobei eine Lage des Wandelements die erste, die zweite und die dritte Fläche sowie eine vierte und eine fünfte Fläche aufweist, wobei jede aus der ersten, der dritten und der fünften Fläche eine im Wesentlichen ebene Hauptfläche bildet, welche einer jeweiligen Platte zugeordnet ist, wobei die erste, die dritte und die fünfte Fläche im Wesentlichen parallel sind, wobei das Paar von Falzen (6) sich an gegenüberliegenden Rändern der ersten Fläche befindet, und wobei die zweite Fläche von dem falzwärtigen Rand der dritten Fläche zu dem jeweiligen Falzrand der ersten Fläche verläuft, und wobei die vierte Fläche von dem falzwärtigen Rand der fünften Fläche zu dem jeweiligen Falzrand der ersten Fläche verläuft, und wobei sich die zweite und die vierte Fläche bei ungefähr 90 Grad zu der ersten, der dritten und der fünften Fläche befinden, sodass sich dann, wenn die Falze (6) um 90 Grad geklappt werden, die zweite und die vierte Fläche mit der ersten Fläche ausrichten, und die dritte und die fünfte Fläche orthogonal von den Rändern der zweiten und der vierten Fläche aus verlaufen, wobei die geklappten Falze (6) in ihrer Gesamtheit auf einer Fläche angeordnet sind, die durch die erste, die zweite und die vierte Fläche gebildet ist.

## Revendications

1. Armoire destinée à un appareil comprenant un élément de paroi formé en thermoformant des feuilles jumelées, ledit élément de paroi (1) comprenant une pluralité de panneaux, lesdits panneaux étant raccordés les uns aux autres par un ou plusieurs bords assemblés où lesdites feuilles jumelées (2, 3) sont en contact pour former un joint (6), et ledit élément de paroi (1) étant dans une condition pliée le long d'un ou de plusieurs joints (6) de sorte que ladite pluralité de panneaux forme une pluralité de parois (8, 9, 10) de ladite armoire, qui est, en tant que groupe, configurée différemment par rapport au moment où ledit élément de paroi est thermoformé de manière initiale, dans lequel au moins l'un de ladite pluralité de panneaux comprend une transition, qui dans la condition pliée forme un coin de l'armoire, **caractérisée en ce qu'**un ou plusieurs desdits joints (6) pliés ne coïncident pas avec le coin entre les parois de ladite armoire qui correspondent aux panneaux assemblés par le joint plié (6) respectif.

2. Armoire selon la revendication 1, dans laquelle ledit joint plié (6) se trouve entre un bord d'un panneau qui forme un bord de la surface (61) contribuant au moins à ladite paroi dudit coin dudit un panneau, et le bord d'un autre panneau qui forme un bord d'une surface (62) selon un angle par rapport à la surface dudit autre panneau contribuant au moins à l'autre paroi dudit coin, ledit élément de paroi (1) plié au niveau d'un joint (6), selon ledit angle, de sorte que ladite surface (62) selon un angle dudit autre panneau contribue à ladite une de ladite paroi dudit coin, et sépare ledit joint plié (6) dudit coin.

3. Armoire selon la revendication 1, dans laquelle chacun desdits joints pliés (6) se trouve entre un bord dudit panneau et un bord d'un autre panneau, et avec ledit élément de paroi dans une condition dépliée, ledit un panneau comprend une surface de paroi extérieure (61) adjacente audit joint, et ledit autre panneau comprend une première surface de paroi extérieure (62) et une seconde surface de paroi extérieure (63), ladite première surface de paroi extérieure (62) étant adjacente audit joint et selon un angle par rapport à ladite surface de paroi extérieure (61) dudit un panneau, et ledit élément de paroi (1) est dans ladite condition pliée avec ladite surface de paroi extérieure (61) dudit un panneau et ladite surface de paroi extérieure (62) dudit autre panneau contribuant à une paroi de ladite armoire, et ladite première surface de paroi extérieure (62) dudit autre panneau séparant ledit joint plié (6) dudit coin.

4. Elément de paroi formé en thermosoudant des feuilles jumelées comprenant un ou plusieurs joints (6) dans lequel lesdites feuilles jumelées sont en contact, ledit élément de paroi étant adapté pour être plié au niveau d'un ou de plusieurs desdits joints (6) pour former une pluralité de parois, au moins l'un desdits joints (6) étant prévu pour être plié selon un angle déterminé à l'usage pour assembler une armoire, et au moins l'une desdites feuilles desdites feuilles jumelées comprenant au moins une première (61) et une seconde surface (62), **caractérisé en ce qu'**au niveau de ou sensiblement au niveau dudit joint (6), une transition située entre lesdites première et seconde surfaces est formée, l'angle de ladite transition étant le complément dudit angle déterminé, de sorte que si ledit joint est plié selon ledit angle déterminé, la première surface (61) et la seconde surface (62) sont alignées sur le joint pour former un plan sensiblement continu sur l'extérieur de ladite armoire.

5. Elément de paroi selon la revendication 4, dans lequel ladite feuille comprenant lesdites première et seconde surfaces, comprend une troisième surface (63), et ladite seconde surface (62) rencontre ladite troisième surface (63) au niveau d'un coin en saillie sensiblement parallèle audit joint (6) mais espacé de celle-ci, ledit coin comprenant ledit angle déterminé de sorte que ladite troisième surface (63) est sensiblement coplanaire avec ladite première surface.

6. Elément de paroi selon la revendication 5 comprenant une paire desdits joints (6), parallèles et espacés, divisant ledit élément de paroi en trois panneaux côte à côte, une feuille dudit élément de paroi comprenant lesdites première, seconde et troisième surfaces et des quatrième et cinquième surfaces, avec chacune desdites première, troisième et cinquième surfaces qui constituent une surface principale sensiblement plane associée avec un panneau respectif, lesdites première, troisième et cinquième surfaces étant sensiblement parallèles, avec ladite paire de joints (6) qui se trouve au niveau des bords opposés de ladite première surface et ladite seconde surface s'étendant à partir du bord vers le joint de ladite troisième surface jusqu'au bord de joint respectif de ladite première surface et ladite quatrième surface s'étendant à partir du bord vers le joint de ladite cinquième surface jusqu'au bord de joint respectif de ladite première surface, et lesdites seconde et quatrième surfaces étant approximativement à 90 degrés desdites première, troisième et cinquième surfaces, de sorte que si lesdits joints (6) sont pliés à 90 degrés, lesdites seconde et quatrième surfaces s'alignent avec ladite première surface et lesdites troisième et cinquième surfaces s'étendent perpendiculairement des bords desdites seconde et quatrième surfaces avec les joints pliés (6) disposés complètement sur une face formée par lesdites première, seconde et quatrième surfaces.
